# EUROPÄISCHE PATENTSCHRIFT

(11) **EP 3 307 170 B1**
(45) Veröffentlichungstag und Bekanntmachung des Hinweises auf die Patenterteilung: **20.01.2021**
(21) Anmeldenummer: 16751465.2
(22) Anmeldetag: 10.06.2016
(51) Int. Cl.: A61B 7/00, A61B 7/04, A61B 5/00

(54) **HUMAN- ODER VETERINÄRDIAGNOSTISCHER KÖRPERSCHALLAUFNEHMER**
HUMAN OR VETERINARY DIAGNOSTIC BODY-BORNE SOUND SENSOR
ENREGISTREUR DE BRUITS CORPORELS DE DIAGNOSTIC HUMAIN OU VÉTÉRINAIRE

(30) Priorität: 12.06.2015 DE 102015109442
(43) Veröffentlichungstag der Anmeldung: 18.04.2018
(73) Patentinhaber: Schwalbe, Hans-Joachim, 35753 Greifenstein-Holzhausen (DE); Franke, Ralf-Peter, 52078 Aachen (DE); Dörner, Peter, 94060 Pocking (DE)
(72) Erfinder: Schwalbe, Hans-Joachim, 35753 Greifenstein-Holzhausen (DE); Franke, Ralf-Peter, 52078 Aachen (DE); Dörner, Peter, 94060 Pocking (DE)
(74) Vertreter: Hebing, Norbert
(86) Internationale Anmeldenummer: PCT/DE2016/100266
(87) Internationale Veröffentlichungsnummer: WO 2016/198049

(56) Entgegenhaltungen:
- CN-Y- 2 686 536
- US-A- 4 736 749
- US-A- 5 852 263
- US-A1- 2006 293 606

## Beschreibung

Die Erfindung bezieht sich auf einen Körperschallaufnehmer zum Aufsetzen auf die Haut eines zu untersuchenden lebenden Körpers mit einem druckempfindlichen Sensorelement zur Körperschallaufnahme, das eine Druckfläche aufweist.

Derartige Körperschallaufnehmer sind bekannt. Sie sind so konstruiert, dass die Druckfläche unmittelbar auf die enthaarte Haut der zu untersuchenden Person aufgesetzt wird, um die Geräusche, die durch Reibung in Gelenken und/oder Rissbildung im Knochen erzeugt werden und als Körperschall vorliegen, aufnehmen zu können.

Diese Geräusche müssen von den Geräuschen diskriminiert werden, die durch Sehnen- und Muskelspannungen, Blutströmungen und der Atmung entstehen. Dies erfolgt durch eine Auswahl geeigneter Prüffrequenzen und einer schallmodenselektiven Auskopplung der Schallsignale.

Als Sensorelement wird ein piezoelektrischer Wandler eingesetzt, der die mechanischen Schwingungen in elektrische Schwingungen transformiert. Der eingesetzte Piezokristall besitzt eine Zylinderform, wobei Schwingungsanregung einerseits in der Achse des Zylinders und andererseits in seinem Radius erfolgt. Bei klinischen Untersuchungen werden die Schwingungen in Axialrichtung analysiert.

Zur Messung des Schallspektrums, das von den oben genannten Geräuschquellen herrührt, wird der Aufnehmer auf die Haut aufgesetzt. Aufgrund des hohen Wassergehalts des menschlichen Unterhautgewebes und der Haut wird der größte Anteil der Schallenergie durch Longitudinalwellen dargestellt.

Die US 2006/0293606 A1 beschreibt eine Sensoranordnung zur Erfassung von Herz- und Atmungstönen. Die Anordnung besteht aus einem Luftkissen, das über einen Schlauch mit einer Sensorkammer verbunden ist, in dem ein piezoelektrischer Sensor (24) angeordnet ist, mit dessen Hilfe der Luftdruck bzw. eine daraus resultierende Luftdruckänderung innerhalb des Luftkissens erfasst wird.

Es besteht generell die Aufgabe, einen möglichst hohen Anteil dieser Energie in den Körperschallaufnehmer einzuleiten, wobei sich gezeigt hat, dass mit dem bisherigen Körperschallaufnehmer, dessen Druckfläche unmittelbar auf die Haut aufgesetzt wird, nicht immer optimale Ergebnisse erzielt werden.

Die Erfindung beruht daher im Speziellen auf der Aufgabe, zu einer besseren Übertragung des Körperschalls auf den Körperschallaufnehmer zu gelangen.

Dies wird erfindungsgemäß dadurch erreicht, dass vor der Druckfläche ein Übertragungskeil zum Übertragen von Körperschall angeordnet ist, wobei der Übertragungskeil eine Kontaktfläche und eine in einem spitzen Winkel dazu verlaufende Koppelfläche aufweist, wobei die Koppelfläche flächig an der Druckfläche anliegt und die Kontaktfläche frei liegt, um in Kontakt mit der Haut gebracht werden zu können.

Durch diese Maßnahme konnten wesentlich bessere Ergebnisse erzielt werden. Dies lässt sich dadurch erklären, dass der longitudinale Anteil des Körperschalls unter bestimmten Winkeln zur Schallquelle emittiert. Bei der Weiterleitung des Schalls durch das menschliche Gewebe wird an jeder Grenzfläche, z. B. Knochen/Muskel, Muskel/Unterhautfettgewebe und Muskelhautfettgewebe/Haut die Schallwelle gebrochen und in einen Longitudinal- und einen Transversalwellenanteil aufgespaltet, wobei die beiden Wellenarten aber unterschiedliche Energieanteile des Primärereignisses beinhalten.

Die Schallleitung von der Schallquelle zum Körperschallaufnehmer ist zwar nicht reproduzierbar und bei jedem Menschen anders. Jedoch ist die Richtung der Longitudinalwelle bei der Übertragung von der Haut über ein auf die Haut aufgetragenes Koppelgel in den Körperschallaufnehmer an einen Winkel gebunden, der in Abhängigkeit des zu untersuchenden Gelenkes und des individuellen Gewebezustandes - z. B. Schichtdicke, Wassergehalt der Schicht - zwischen 40° und 70° variiert.

Der Körperschall sollte aber möglichst in Axialrichtung des Körperschallaufnehmers auf diesen treffen. Um die Übertragungsverluste des Schalls zu minimieren, wird der erfindungsgemäße Übertragungskeil genutzt, wobei der Winkel zwischen den beiden Flächen zwischen 20°-50° für eine optimale Schallübertragung sinnvoll ist. Dieser Winkelbereich unterstützt die Selektion der nutzbaren Schallsignale aus dem gesamten Schallumfeld.

Vorzugsweise besteht der Übertragungskeil aus einem Metall bzw. aus Aluminium. Andere Materialien sind ebenfalls physikalisch nutzbar, aber mit einer größeren Dämpfung der Signalamplitude verbunden.

Da die Oberfläche der Haut an keiner Stelle eben ist und die Nachgiebigkeit des menschlichen Gewebes größer ist als die des Übertragungskeils, würde unter diesen Bedingungen eine ebene Kontaktfläche zur Haut zu ungleicher Schallübertragung zwischen Haut und Übertragungskeil führen. Zur gleichmäßigen Schallübertragung ist die Kontaktfläche zumindest in einer Ebene konvex gewölbt, wobei die Sekantenfläche der Wölbung einen spitzen Winkel mit der Koppelfläche einschließt. Die Wölbung kann von der Mantelfläche eines Zylindersegments oder vorzugsweise von der Oberfläche einer Kalotte gebildet werden. Die Sekantenfläche ist die eben verlaufende Basisfläche der jeweiligen Wölbung, die als Referenzfläche zur Bestimmung des Keilwinkels des Übertragungskeiles dient.

Wenn die Kontaktfläche von einer Kalotte gebildet ist, liegt in jeder Richtung eine konvexe Kontur vor. Damit kann man in gewissen Grenzen die unterschiedlichen Abstrahlwinkel von verschiedenen Körpergeweben ausgleichen.

Der Körperschallaufnehmer wird vorzugsweise derart aufgebaut, dass dieser ein Gehäuse mit einer Stirnfläche aufweist, wobei in die Stirnfläche ein dazu schräg verlaufender Aufnahmekanal einmündet, in dem das Sensorelement derart aufgenommen ist, dass seine Druckfläche zur Stirnfläche weist und wobei der Übertragungskeil in der Kanalmündung befestigt ist.

Das Gehäuse besteht vorzugsweise aus Kunststoff, wobei sich an der Wand des Aufnahmekanals im Bereich der Mündung ein umlaufender Kragen befindet, der in einer umlaufenden Nut des Übertragungskeiles liegt.

Das Gehäuse selbst kann aus Kunststoff bestehen, was leichter herzustellen ist. Außerdem ermöglicht es, wegen der Nachgiebigkeit des Kunststoffes, den Übertragungskeil in die Mündung einzuknüpfen.

Der Körperschallaufnehmer wird bei einer Messung mittels Unterdruck auf die Haut gepresst. Dazu besitzt der Körperschallaufnehmer eine ringförmige Saugglocke, die mit ihrem Innenrand an einem mittleren Bund der Mantelfläche des Gehäuses luftdicht befestigt ist. Durch die Gehäusewand verläuft ein Verbindungskanal, der die Öffnung des Gehäuses ober- und unterhalb des Bundes miteinander verbindet.

Die Öffnung unterhalb des Bundes befindet sich unterhalb der Saugglocke. Die Öffnung oberhalb des Bundes wird an eine Unterdruckquelle angeschlossen, so dass die Saugglocke evakuiert werden kann, und dadurch der Aufnehmer gegen die Haut der zu untersuchenden Person gedrückt wird.

Im Folgenden wird anhand eines Ausführungsbeispiels die Erfindung näher erläutert.

Dazu zeigen:
- Fig. 1: eine Seitenansicht eines erfindungsgemäßen Körperschallaufnehmers mit einem Übertragungskeil,
- Fig. 2: einen Schnitt durch das Gehäuse des Körperschallaufnehmers und einer Saugglocke in einer ersten Ebene,
- Fig. 3: einen Schnitt durch das Gehäuse gemäß Fig. 2 in einer zweiten Ebene mit einem eingesetzten Sensor und
- Fig. 4: eine perspektivische Darstellung des Übertragungskeils.

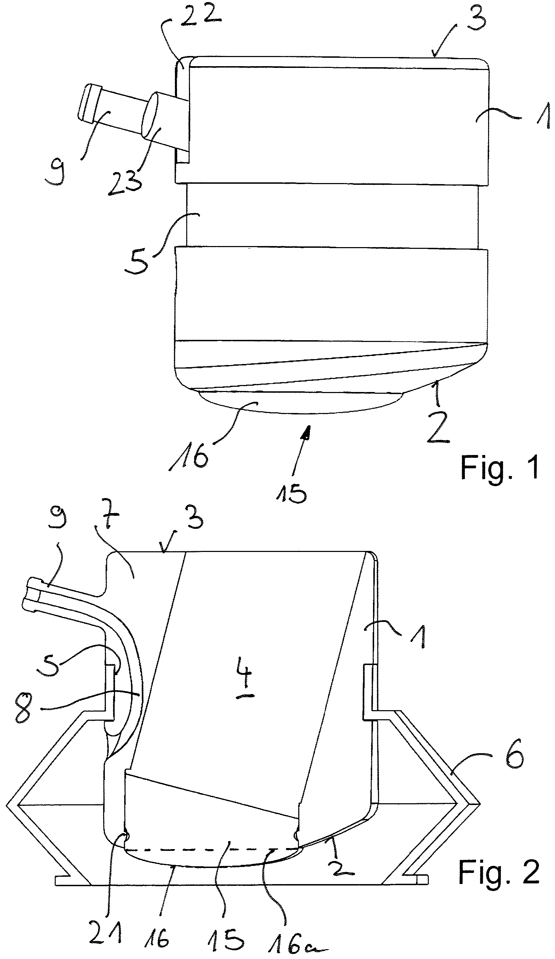

Wie insbesondere der Fig. 2 zu entnehmen ist, besteht ein erfindungsgemäßer Körperschallaufnehmer aus einem zylindrischen Gehäuse 1 mit einer unteren und einer oberen Stirnseite 2, 3. Zwischen den beiden Stirnseiten erstreckt sich ein Aufnahmekanal 4, der schräg zu der unteren und oberen Stirnseite 2, 3 verläuft.

Im mittleren Bereich der Mantelfläche des Gehäuses 1 befindet sich ein Bund, der von einer umlaufenden Nut 5 gebildet wird, in der eine ringförmige Saugglocke 6 mit ihrem inneren Rand eingeknüpft ist. Die Saugglocke 6 erstreckt sich zur unteren Stirnseite 2.

Durch die Wand 7 des Gehäuses 1 verläuft ein Verbindungskanal 8, der eine Öffnung unterhalb der Nut 5, also innerhalb der Saugglocke 6, mit einer Öffnung oberhalb der Nut 5 verbindet, wobei diese Öffnung in einem Nippel 9 zum Aufstecken eines Saugschlauches (nicht dargestellt) ausgeformt ist.

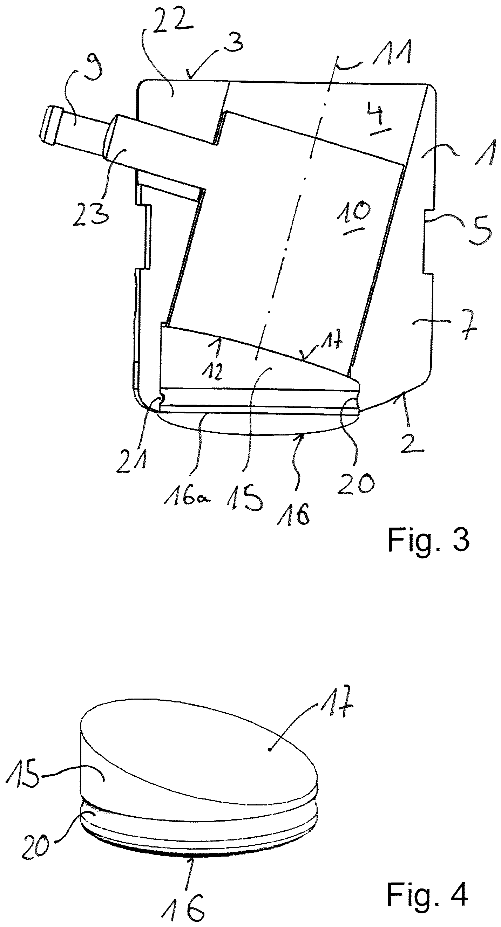

Die Fig. 3 zeigt einen Schnitt durch das Gehäuse 1, wobei die Schnittebene gegenüber der Schnittebene in der Fig. 2 gedreht ist. Fig. 3 zeigt außerdem ein Sensorelement 10, das in der Fig. 2 aus Übersichtsgründen nicht dargestellt ist. Das Sensorelement 10, das in den Aufnahmekanal 4 passend eingesetzt ist, ist durch einen Piezokristall realisiert, dessen Messachse 11 in der Achse des zylindrischen Aufnahmekanals 4 liegt. Daher verläuft die Druckfläche 12 des Sensorelements 10, die zu der Messachse 11 senkrecht verläuft, schräg zur unteren Stirnseite 2 des Gehäuses 1.

Zwischen der Druckfläche 12 und der unteren Stirnseite 2 befindet sich ein Übertragungskeil 15 mit einer kalottenförmigen Kontaktfläche 16 auf einer Kalotte und einer Koppelfläche 17, die plan ist und flächig an der Druckfläche 12 anliegt. Die Koppelfläche 17 und Druckfläche 12 werden mit einem schallleitenden Kleber, z. B. einem Cyanacrylat-Kleber, flächig miteinander verbunden.

Der Winkel zwischen der Koppelfläche 17 und der parallel zur Koppelfläche 17 verlaufenden Sekantenfläche 16a der Kalotte liegt je nach Ausführung wahlweise zwischen 20° und 50°, um eine optimale Körperschallübertragung bei einer Diagnose zu erreichen. Die Sekantenfläche 16a ist die eben verlaufende Fläche, die von der Wölbung der Kalotte gleichmäßig überspannt wird. Sie dient als Referenzfläche, um den Winkel des Übertragungskeils 15 zu bestimmen.

Wie der Fig. 4 zu entnehmen ist, weist der Übertragungskeil 15 eine umlaufende, im Querschnitt runde Nut 20 auf, die in einen entsprechenden umlaufenden Kragen 21 am unteren Ende des Aufnahmekanals 4 eingeknöpft ist.

In der Gehäusewand befindet sich - ausgehend vom oberen Rand des Gehäuses 1 ein Schlitz 22, durch den ein elektrischer Anschluss 23 für das Sensorelement 10 nach außen geführt ist.

In der Fig. 1 sind die vorgenannten Elemente noch einmal in einer perspektivischen Form zu erkennen. Aus dem Gehäuse 1 ragen seitlich sowohl der Nippel 5 für den Druckluftanschluss als auch der elektrische Anschluss 23 für das Sensorelement 10 hervor.

Die untere Stirnseite 2 ist passend zur kalottenförmigen Ausformung der Kontaktfläche 16 des Übertragungskeiles 15 gerundet geformt, so dass keine Übergangskanten entstehen, die die Haut der zu untersuchenden Person verletzen könnte.

### Bezugszeichenliste

- 1: Gehäuse
- 2: untere Stirnseite
- 3: obere Stirnseite
- 4: Aufnahmekanal
- 5: umlaufende Nut

- 6: Saugglocke
- 7: Wand
- 8: Verbindungskanal
- 9: Nippel
- 10: Sensorelement

- 11: Messachse
- 12: Druckfläche
- 15: Übertragungskeil

- 16: Kontaktfläche
- 16a: Sekantenfläche
- 17: Koppelfläche
- 20: runde Nut

- 21: Kragen
- 22: Schlitz
- 23: elektrische Anschluss

## Patentansprüche

1. Human- oder veterinärdiagnostischer Körperschallaufnehmer zum Aufsetzen auf die Haut eines zu untersuchenden lebenden Körpers mit einem druckempfindlichen Sensorelement (10) zur Körperschallaufnahme, das eine Druckfläche (12) aufweist, **dadurch gekennzeichnet, dass** vor der Druckfläche (12) ein Übertragungskeil (15) zum Übertragen von Körperschall angeordnet ist, wobei der Übertragungskeil (15) eine Kontaktfläche (16) und eine in einem spitzen Winkel dazu verlaufende Koppelfläche (17) aufweist, wobei die Koppelfläche (17) flächig an der Druckfläche (12) anliegt und die Kontaktfläche (16) frei liegt, um in Kontakt mit der Haut gebracht werden zu können.

2. Körperschallaufnehmer nach Anspruch 1, **dadurch gekennzeichnet, dass** der Übertragungskeil (15) aus einem Metall besteht.

3. Körperschallaufnehmer nach Anspruch 2, **dadurch gekennzeichnet, dass** der Übertragungskeil (15) aus Aluminium besteht.

4. Körperschallaufnehmer nach Anspruch 1, **dadurch gekennzeichnet, dass** die Kontaktfläche (16) in zumindest einer Ebene konvex gewölbt ist, wobei die Sekantenfläche (16a) der Wölbung einen spitzen Winkel mit der Koppelfläche (17) einschließt.

5. Körperschallaufnehmer nach Anspruch 4, **dadurch gekennzeichnet, dass** die Kontaktfläche (16) von einer Kalotte gebildet ist.

6. Körperschallaufnehmer nach einem der vorhergehenden Ansprüche, **dadurch gekennzeichnet, dass** dieser ein Gehäuse (1) mit einer Stirnfläche aufweist, wobei in die Stirnfläche ein dazu schräg verlaufender Aufnahmekanal (4) einmündet, in dem das Sensorelement (10) derart aufgenommen ist, dass seine Druckfläche (12) zur Stirnfläche weist, und wobei der Übertragungskeil (15) in der Kanalmündung befestigt ist.

7. Körperschallaufnehmer nach Anspruch 6, **dadurch gekennzeichnet, dass** das Gehäuse (1) vorzugsweise aus Kunststoff besteht, wobei sich an der Wand des Aufnahmekanals (4) im Bereich der Mündung ein umlaufender Kragen (21) befindet, der in einer umlaufenden Nut (20) des Übertragungskeiles (15) liegt.

8. Körperschallaufnehmer nach einem der vorhergehenden Ansprüche, **dadurch gekennzeichnet, dass** dieser eine ringförmige Saugglocke (6) besitzt, die mit ihrem Innenrand an einen mittleren Bund der Mantelfläche des Gehäuses (1) luftdicht befestigt ist und dass durch die Gehäusewand ein Verbindungskanal (8) verläuft, der Öffnungen des Gehäuses (1) oberhalb und unterhalb des Bundes miteinander verbindet.

## Claims

1. A human or veterinary diagnostic structure-borne sound sensor for placing on the skin of a living body to be examined, comprising a pressure-sensitive sensor element (10) for registering structure-borne sound, the sensor having a pressure surface (12), **characterised in that** a transmission wedge (15) for transmitting structure-borne sound is arranged in front of the pressure surface (12), wherein the transmission wedge (15) has a contact surface (16) and a coupling surface (17) extending at an acute angle thereto, wherein the coupling surface (17) is entirely in contact with the pressure surface (12) and the contact surface (16) is exposed in order to be able to be brought into contact with the skin.

2. The structure-borne sound sensor according to claim 1, **characterised in that** the transmission wedge (15) consists of a metal.

3. The structure-borne sound sensor according to claim 2, **characterised in that** the transmission wedge (15) consists of aluminium.

4. The structure-borne sound sensor according to claim 1, **characterised in that** the contact surface (16) is convexly arched in at least one plane, wherein the secant surface (16a) of the arch forms an acute angle with the coupling surface (17).

5. The structure-borne sound sensor according to claim 4, **characterised in that** the contact surface (16) is formed by a calotte.

6. The structure-borne sound sensor according to one of the preceding claims, **characterised in that** the sensor comprises a housing (1) with a front face, wherein an accommodating channel (4), extending obliquely to the front face, ends therein, the sensor element (10) being contained in the accommodating channel such that its pressure surface (12) points towards the front face, and wherein the transmission wedge (15) is fixed in the mouth of the channel.

7. The structure-borne sound sensor according to claim 6, **characterised in that** the housing (1) preferably consists of plastic, wherein there is a circumferential collar (21) on the wall of the sensing channel (4) in the region of the mouth, the collar lying in a circumferential groove (20) of the transmission wedge (15).

8. The structure-borne sound sensor according to one of the preceding claims, **characterised in that** the sensor has an annular suction cup (6), which is fixed with its inner rim in an air-tight manner to a central flange of the outer surface of the housing (1), and **in that** a connecting channel (8) extends through the housing wall, the connecting channel connecting openings of the housing (1) above and below the flange with each other.

## Revendications

1. Détecteur de sons corporels pour diagnostic humain ou vétérinaire à poser sur la peau d'un corps vivant examiné avec un élément de détection sensible à la pression (10) pour l'enregistrement de sons corporels, qui comporte une surface de compression (12), **caractérisé en ce qu'**une cale de transmission (15) est disposé devant la surface de compression (12) pour transmettre des sons corporels, sachant que la cale de transmission (15) comporte une surface de contact (16) et une surface de couplage (17) passant à cet effet dans un angle aigu, sachant que la surface de couplage (17) est entièrement appliquée sur toute la surface de compression (12) et la surface de contact (16) est libre pour pouvoir être mise en contact avec la peau.

2. Détecteur de sons corporels selon la revendication 1, **caractérisé en ce que** la cale de transmission (15) est composé de métal.

3. Détecteur de sons corporels selon la revendication 2, **caractérisé en ce que** la cale de transmission (15) est en aluminium.

4. Détecteur de sons corporels selon la revendication 1, **caractérisé en ce que** la surface de contact (16) est bombée de façon convexe dans au moins un plan, sachant que la face sécante (16a) du bombement forme un angle aigu avec la face de couplage (17).

5. Détecteur de sons corporels selon la revendication 4, **caractérisé en ce que** la surface de contact (16) est formée par une calotte.

6. Détecteur de sons corporels selon l'une quelconque des revendications précédentes, **caractérisé en ce que** celui-ci comporte un boîtier (1) avec une face avant, sachant que dans la face avant débouche un conduit de réception (4) passant de façon inclinée à cet effet dans lequel l'élément de détection (10) est logé de telle manière que sa surface de compression (12) est tournée vers la face avant et sachant que la cale de transmission (15) est fixée dans l'embouchure de conduit.

7. Détecteur de sons corporels selon la revendication 6, **caractérisé en ce que** le boîtier (1) est de préférence en matière plastique, sachant que sur la paroi du conduit de réception (4) se trouve une collerette (21) périphérique dans la zone de l'embouchure, qui se situe dans une rainure périphérique (20) de la cale de transmission (15).

8. Détecteur de sons corporels selon l'une quelconque des revendications précédentes, **caractérisé en ce que** celui-ci possède une cloche d'aspiration (6) de forme annulaire, qui est fixée étanche à l'air à son bord intérieur sur un collet central de la surface d'enveloppe du boîtier (1) et **en ce qu'**un conduit de liaison (8) passe à travers la paroi de boîtier, qui relie entre elles les ouvertures du boîtier (1) au-dessus et en dessous du collet.
